# EUROPEAN PATENT APPLICATION

(11) **EP 4 702 937 A1**
(43) Date of publication of application: **04.03.2026**
(21) Application number: 25198672.5
(22) Date of filing: 28.08.2025
(51) Int. Cl.: A61B 17/34, A61B 17/00, A61M 39/06

(54) **TROCAR WITH CLEANING AND SMOKE EVACUATION**

(30) Priority: 29.08.2024 WO PCT/CN2024/115408
(71) Applicant: Covidien LP, Mansfield, MA 02048 (US)
(72) Inventor: Jin, Tingting, Mansfield, 02048 (US); Sun, Chengjun, Mansfield, 02048 (US)
(74) Representative: Gray, James

(57) **Abstract**

A trocar with cleaning and smoke evacuation functions is provided for use during endoscopic surgical procedures. The trocar includes a housing and an elongated shaft with channels and an access valve at the distal tip. The trocar is fitted with a multiport valve that alternates between an inlet for cleaning and an outlet for smoke filtering.

## Description

### CROSS-REFERENCE TO RELATED APPLICATIONS

This application claims the benefit of International Patent Application Serial No. PCT/CN2024/115408, filed on August 29, 2024, the entire content of which is incorporated herein by reference.

### FIELD

The disclosure is generally related to a medical device for use in minimally invasive surgical procedures, such as endoscopic and laparoscopic procedures, and more particularly to a trocar with a cleaning and smoke evacuation system.

### BACKGROUND

During laparoscopic and robotic surgery, trocars are utilized for creating small incisions through which the surgery is performed. Trocar tubes or cannulas are extended into and left in place in the abdominal wall to provide access points for endoscopic surgical instruments inserted through the trocar. Instruments inserted through trocars include forceps, graspers, cutters, applicators, endoscopes, laparoscopes, and other surgical, therapeutic and visualization tools. These can be used during arthroscopy, laparoscopy, gastroentroscopy, bronchoscopy, and other types of minimally invasive procedures.

In particular, a camera or laparoscope is often inserted through a trocar to permit the visual inspection and magnification of the body cavity. Within the surgical site, lenses on these instruments can become covered or blocked by condensation, body fluids, and smoke produced by surgical tools doing cutting, cauterizing, or ablating. Removing the tool from the trocar to clean and then reinsert it increases a risk of infection at the site as well as increasing surgery time, cost, and the amount of time a patient remains under anesthesia. There is a need for a trocar system that can effectively clean a portion of an instrument such as a lens in vivo.

### SUMMARY

The techniques of this disclosure generally relate to a trocar with a cleaning and smoke evacuation system for use during endoscopic surgical procedures such as arthroscopy, laparoscopy, gastroentroscopy, bronchoscopy, and others. According to an embodiment, a trocar includes a multiport valve that alternates between lens cleaning and smoke filtering functions, to improve cleaning and reduce the impact of smoke contamination during a surgical procedure.

In an embodiment, a surgical access device includes a trocar fitted with a multiport valve. The trocar includes a proximal housing with a first port and a second port and an elongated shaft extending from the proximal housing to a distal end. First and second channels are formed in the elongated shaft, and an access valve is attached at the distal end of the elongated shaft. The multiport valve is fitted to the first port and includes an inlet, an outlet, a central branch, and a valve stem. The valve stem rotates between a first position exposing the central branch to the inlet and blocking the outlet, and a second position exposing the central branch to the outlet and blocking the inlet. The access valve has a flexible face with four petals divided by first and second intersecting slits. The first slit is positioned in a first recess in the face and the second slit is positioned in a second recess in the face. These recesses form openings for gas exit when the access valve is flexed open.

In an embodiment, the first channel is bidirectional. When the multiport valve is in the first position, a fluid flow path for cleaning solution is formed through the first channel in a distal direction. When the multiport valve is in the second position, a gas exit path is formed through the first channel in a proximal direction.

In an embodiment, a surgical access device includes a trocar, a multiport valve, and a filter. The trocar has a proximal housing with a valve port and a suction port, and an elongated shaft extending from the proximal housing to a distal end. A central lumen passes through the elongated shaft. The trocar also has a cannula fitted over the elongated shaft, and a first channel and a second channel between the elongated shaft and the cannula. An access valve is attached at the distal end of the elongated shaft. The multiport valve is fitted to the valve port, and it includes an inlet, an outlet, a central branch, and a valve stem. The valve stem rotates between a first position exposing the central branch to the inlet and blocking the outlet, and a second position exposing the central branch to the outlet and blocking the inlet. The filter is tightly mated with the outlet of the multiport valve. The access valve has a face with petals divided by first and second intersecting slits. The petals flex to form gas pathway chutes in response to passage of a medical instrument such as an endoscope through the access valve in a distal direction.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a surgical system including a medical instrument passing through a trocar according to an embodiment of the present disclosure.
FIG. 2 is a lower perspective view of a trocar body according to an embodiment of the present disclosure.
FIG. 3 is a partial side view of a trocar with a multiport valve according to an embodiment of the present disclosure.
FIG. 4 is a perspective view of a component of the multiport valve of FIG 3.
FIG. 5 is a cut-away cross-sectional view of the multiport valve of FIG. 3 in a first position for instrument cleaning.
FIG. 6 is a cut-away cross-sectional view of the multiport valve of FIG. 3 in a second position for smoke evacuation and filtering.
FIG. 7 is a mixed cross-sectional and perspective view of a medical instrument and trocar with a trocar seal in a closed position for instrument cleaning, according to an embodiment of the present disclosure.
FIG. 8 is a perspective view of the medical instrument and trocar of FIG. 7 with the trocar seal in an open position for smoke evacuation and filtering.
FIG. 9 is a perspective view of a trocar seal according to an embodiment of the present disclosure.
FIG. 10 is a bottom view of the trocar seal of FIG. 9.
FIG. 11 is a top perspective view of the trocar seal of FIG. 9.

### DETAILED DESCRIPTION

The techniques of this disclosure generally relate to a trocar with a cleaning and smoke evacuation system for use during endoscopic surgical procedures such as arthroscopy, laparoscopy, gastroentroscopy, bronchoscopy, and others. According to an embodiment, a trocar includes a multiport valve that alternates between lens cleaning and smoke filtering functions, to improve cleaning of a surgical instrument and reduce the impact of smoke contamination during a surgical procedure. This trocar can filter smoke out of the surgical site or pneumoperitoneum as well as clean the instrument in vivo. This can reduce surgery time and contribute to the postoperative recovery of patients.

According to an embodiment of the disclosure, a trocar with both lens cleaning and smoke filtering includes a multiport valve that alternately connects the trocar to an inlet for a cleaning solution such as saline and an outlet for smoke filtering. During cleaning, the multiport valve opens access to the cleaning solution and block the filter cartridge, so that the filter does not get wet. Between cleaning intervals, the multiport valve is turned to block the cleaning solution and expose the filter end. Smoke inside the body cavity is drawn out through the valve exit port and through the filter under the influence of the pressure difference between the inside and outside of the trocar. This function improves the surgical view and reduces lens cleaning time, while also maintaining the cleanliness of the operating room air for the safety of the medical caregivers.

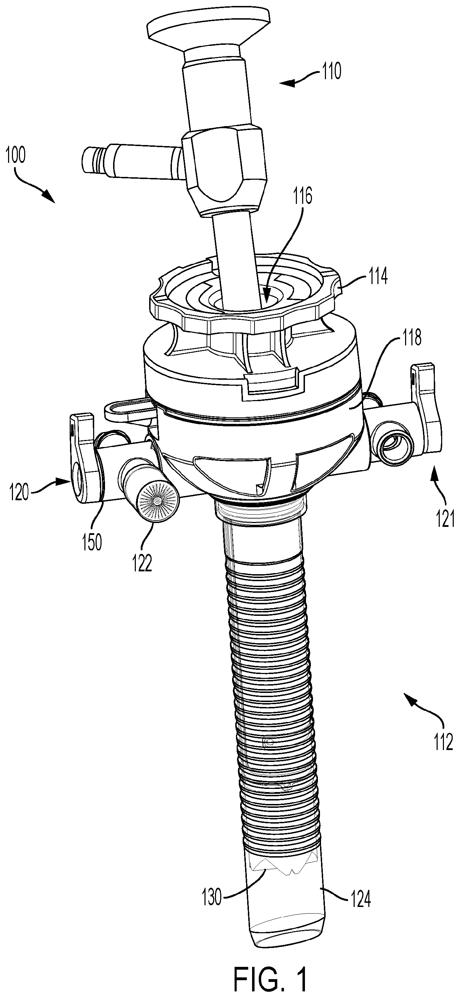

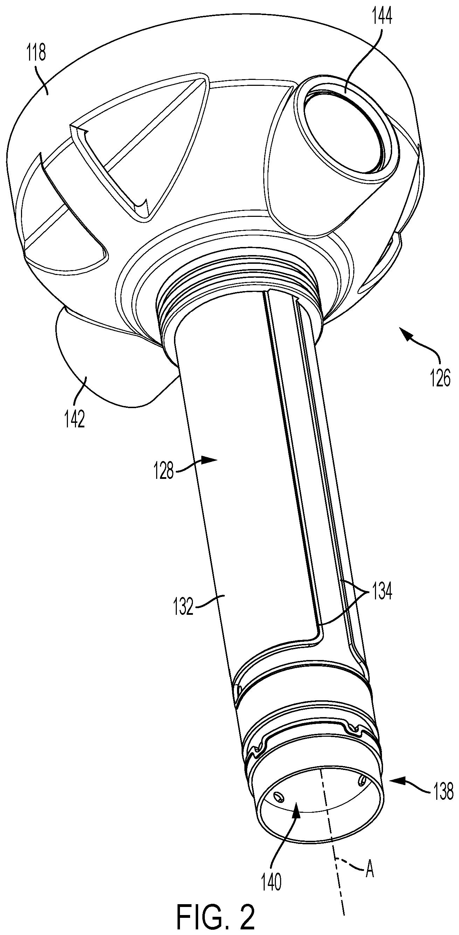

A surgical access system 100 according to an embodiment is depicted in Figures 1 and 2. The system 100 includes a medical instrument 110 inserted through a trocar 112. In use, the trocar 112 is inserted through a small incision in the patient's skin to provide access to internal body cavities and tissue. The trocar 112 includes a centering assembly 114 at the upper, proximal end, and the instrument 110 is inserted through a passage 116 in the proximal centering assembly 114. The trocar 112 includes a body 126 with a proximal housing 118 and an elongated shaft 128. The centering assembly 114 is seated in the proximal housing 118. The proximal housing also includes first and second ports or receptacles 142, 144 on opposite sides of the proximal housing 118. The elongated shaft 128 extends from the proximal housing 118 to a distal end or tip 138. The elongated shaft 128 has an open interior, forming a lumen 140 through the shaft. Referring to Figure 1, an access valve or seal 130 is attached to the distal tip 138 of the elongated shaft 128, and the shaft 128 is inserted into an outer cannula 124.

As shown in Figure 1, the trocar 112 with instrument cleaning and smoke filtering includes a multiport valve 120 connected to a filter 122. The multiport valve includes a valve body 150 fitted to one of the ports 142, 144 on the proximal housing 118 of the trocar, and a suction valve 121 is fitted to the opposite port. The suction valve is used during cleaning, as described in more detail below. The suction valve 121 may have the same structure as the multiport valve 120, but is not attached to the filter 122.

During use of the system 100 shown in Figure 1, the trocar 112 maintains an access point to the surgical site, and the instrument 110 is inserted through the trocar 112 and through the distal seal 130 into the surgical site. While the seal 130 is open, the multiport valve 120 is positioned to connect the trocar to the filter 122, to pass smoke from the surgical site through the filter 122 and return filtered air to the ambient room. When the instrument 110 needs to be cleaned, it is withdrawn through the seal 130 into the elongated shaft 128 of the trocar, and the multiport valve 120 is then turned to block the filter 122. In this position, the valve 120 opens access to a cleaning solution (such as a saline bag) which is circulated through the trocar to clean the instrument 110, without wetting the filter 122. A suction source attached to the suction valve 121 draws the cleaning solution out of the trocar. When cleaning is complete, the valve 120 is returned to the filter position, and the cleaned medical instrument 110 can then be advanced distally back through the seal 130 to resume operation. The valve 120 thus protects both the saline solution (sending it through the trocar without having it escape through the filter 122) and protects the filter (keeping the filter media inside dry and not getting saturated with the saline solution, which could cause the filter to malfunction).

Referring to Figure 2, the elongated shaft 128 also includes channels or slots 134 formed in an outer surface 132 of the shaft. The channels 134 run longitudinally along the shaft, parallel with the longitudinal axis A of the shaft. During the cleaning operation, cleaning solution flows into the trocar through a first channel in a distal direction and then exits the trocar through a second channel in a proximal direction, as will be described in more detail below. During smoke removal, smoke particles follow a reverse path through the first channel, moving in a proximal direction back toward the valve 120 and out through the filter 122.

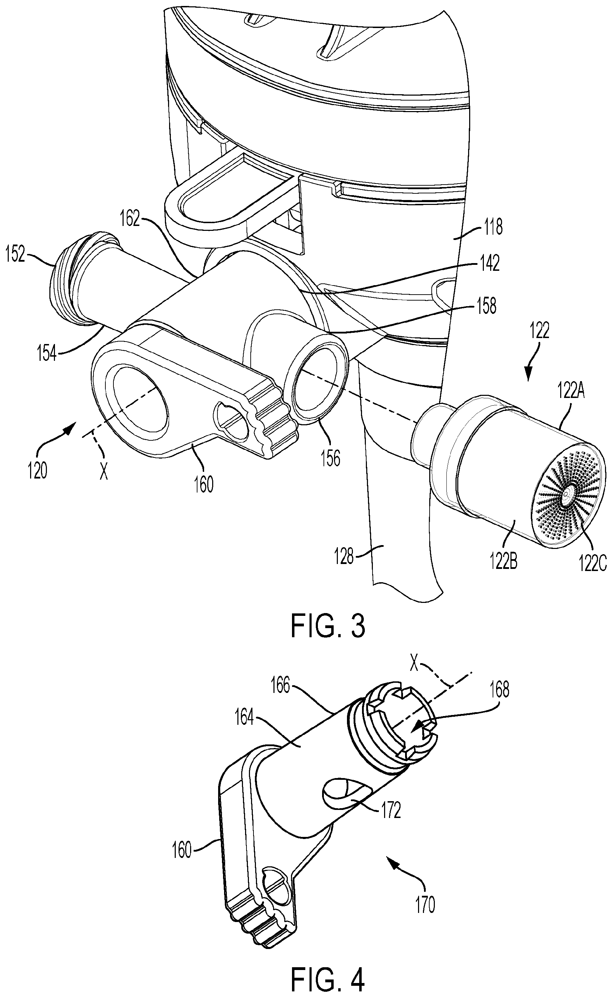

Turning to Figures 3 and 4, an embodiment of the multiport valve 120 is shown. In an embodiment, the multiport valve 120 has a valve body with three branches - a first branch 154, a second branch 158, and a central branch 162 between the first two branches. The first branch 154 includes an inlet port 152 that is connected to a source of cleaning solution such as saline or sterile water, or another therapeutic liquid. The second branch 158 includes an outlet port 156 that is connected to the filter 122. The valve 120 is mounted to the proximal housing 118 by fitting the central branch 162 to the port 142 on the housing 118.

The valve 120 also includes a handle 160 that rotates about axis X. This axis X is aligned with the bore of the central branch 162. Turning the handle 160 to a first position exposes the central branch 162 to the saline inlet 152 and blocks the outlet port 156 and filter 122. Turning the handle 160 to an opposite second position exposes the central branch 162 to the outlet port 156 and filter 122, and blocks the inlet 152. In the embodiment shown, the first and second branches 154, 158 of the valve are on opposite sides of the central branch 162, and the handle is turned 180 degrees between the two positions. In other embodiments, the branches could be arranged differently on the valve body, such as 90 degrees apart.

The filter 122 includes a housing 122A with a radial array of openings 122C at one end. Inside the housing 122A is a filter medium or media 122B which will be described in more detail below. The filter medium 122B is put inside the housing 122A, and a cover can be assembled tightly with housing by ultrasonic welding (adding welding strip feature on the house or cover interface), or by glue applied between the cover and the housing. The housing 122A fits securely into the second branch 158, such as by a friction fit, glue, latch, threads, or other appropriate mechanism.

The handle 160 of the valve 120 attaches to a valve core 170, as shown in Figure 4. The valve core 170 includes a stem 164 with an interior channel 168 that aligns with the central branch 162 along axis X. The stem 164 includes a solid side wall 166 opposite an opening 172. Turning the handle 160 aligns the opening 172 with the first or second branch of the valve and blocks the other branch with the side wall 166. As the handle 160 turns, the interior channel 168 remains aligned with the central branch 162, to connect the central branch with either the first or second branch depending on how the handle is turned.

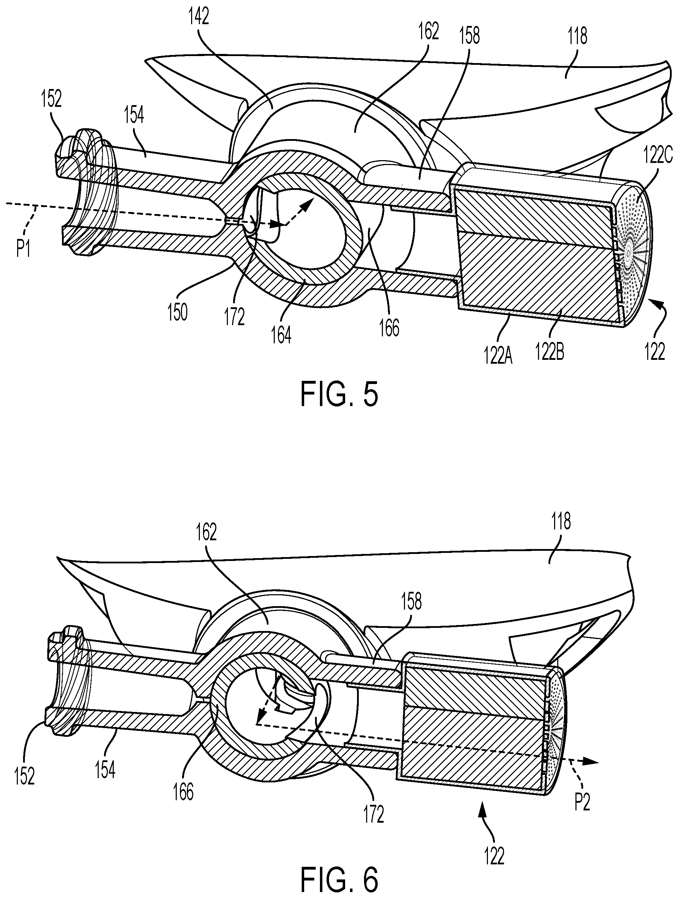

The two active positions of the valve 120 are shown in more detail in Figures 5-6. In Figure 5, the valve 120 is in a first position that opens the trocar to the inlet 152. The handle is turned to rotate the valve stem 164 to align the opening 172 with the first branch 154. This creates a fluid flow path P1 from the inlet port 152 through the first branch 154, through the opening 172, through the interior channel of the valve stem and the central branch 162 of the valve, and into the trocar. The central branch of the multiport valve communicates with the first channel in the elongated shaft of the trocar (such as channel 134 shown in Figure 1). When the inlet port 152 is connected to a cleaning solution supply, cleaning solution can then be flushed into the trocar 112 to clean the medical instrument. In this position, the side wall 166 of the valve stem blocks the second branch 158 and thus prevents liquid from entering the filter 122.

In Figure 6, the valve 120 is in a second position that opens the trocar to the outlet. The handle is turned to rotate the valve stem 164 to align the opening 172 with the second branch 158. This creates a gas exit path P2 from the trocar, through the central branch 162 and the channel of the valve stem, through the second branch 158 and into the filter 122. When the access valve or seal 130 (in Figure 1) of the trocar is pushed open by the medical instrument, this creates a gas escape route from the surgical site through the path P2 to the filter 122, allowing filtered air to exit into the ambient environment. In this position, the side wall 166 of the valve stem blocks the inlet 152, preventing liquid from entering the filter and preventing smoke or gas from the surgical site from entering the ambient environment without first passing through the filter.

When the handle 160 is positioned between the first and second positions, (such as pointing the handle 160 vertically in Figure 3, instead of horizontally), the valve 120 blocks both the first branch 154 and the second branch 158, closing access to the central branch 162 and the trocar. This may be referred to as the closed position of the valve 120. In the closed position, the side wall of the valve stem blocks both the branches 154 and 158 and the opening 172 of the valve stem is facing upward (in Figures 5 and 6). In this position, the opening 172 is not aligned with either the inlet or outlet, so there is no fluid or gas pathway through the valve 120. As an example, closing the valve 120 is useful at the beginning of a surgical procedure, when the trocar 112 is not performing suction, cleaning, or smoke filtering. When the surgeon uses the trocar to create a passage, the valves 120 and 121 should remain closed to facilitate the establishment of pneumoperitoneum.

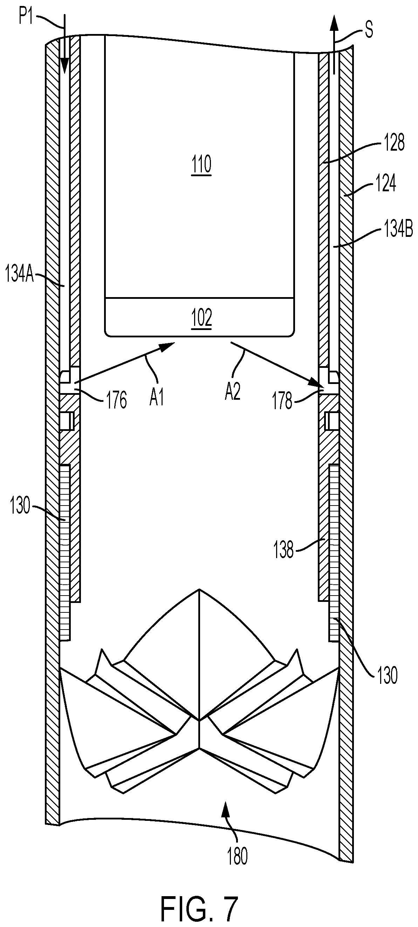

Operation of the trocar to conduct cleaning and smoke filtering will be described in more detail with reference to Figures 7-8. Figure 7 shows the access valve or seal 130 attached at the distal tip 138 of the elongated shaft 128. In Figure 7, a lower distal face 180 of the access valve 130 is show in perspective view, and the remainder of the access valve, trocar, and cannula are shown in cross-section. The trocar shaft 128 and access valve 130 are inserted inside the outer cannula 124. The access valve 130 extends past the elongated shaft 128 at the distal end 138 and contacts the interior wall of the outer cannula 124, forming a peripheral seal. When the access valve 130 is closed, it seals the interior of the trocar from the surgical site below.

Proximally of the access valve 130, the channels 134 form gaps between the elongated shaft 128 and the outer cannula 124. Two channels 134A, 134B are shown in Figure 7. These channels can be formed as slots or grooves in the outer surface of the elongated shaft (as shown in Figure 2), or as slots or grooves in the interior surface of the outer cannula, or as gaps between the two components. The channels 134A, 134B run longitudinally along the trocar, connected at the proximal end to the housing 118 and the ports 142, 144. At the distal end of channel 134A, a first opening 176 connects the first channel 134A to the interior lumen of the trocar, and at the distal end of the second channel 134B a second opening 178 connects the second channel 134B to the interior lumen of the trocar.

In Figure 7, the instrument 110 has been withdrawn inside the trocar for cleaning. In this position, the instrument 110 has been retracted in a proximal direction away from the surgical site and back through the access valve 130. The access valve 130 closes, sealing the instrument 110 inside the central lumen of the trocar. The fluid flow path P1 (from Figure 5) extends through the first channel 134A, through the first opening 176, and into the trocar. When saline is supplied through this path P1, it sprays onto the instrument as shown by arrow A1. The distal end of the instrument may have a distal component 102 (such as a lens, light emitter, opening, sensor, or tool) which can be cleaned by this saline spray. The spray then follows arrow A2 and into the second channel 134B through the second opening 178. The saline is drawn out of the trocar through channel 134B by a suction force shown by arrow S. The suction source, such as a vacuum pump, is connected to the trocar through the suction valve 121 (Figure 1).

When the multiport valve 120 is open to the saline inlet and suction is applied through the suction valve 121, the saline or other cleaning solution is thus drawn into the trocar in a distal direction through the path P1 and across the distal end of the medical instrument 110 to clean the lens 102 or other element there. The cleaning solution is then continued to be drawn back out of the trocar in a proximal direction through channel 134B and out through the suction valve 121. This sends clean fluid into the trocar to clean the instrument 110 and withdraws the contaminated fluid out of the trocar. Particles that have accumulated on the lens 102, such as particles of smoke, body fluid, tissue, or other debris, are cleared from the instrument 110 without having to remove the instrument from the trocar. This cleaning is done at the distal end of the trocar within the space sealed by the access valve 130, while the trocar is still in place in the patient.

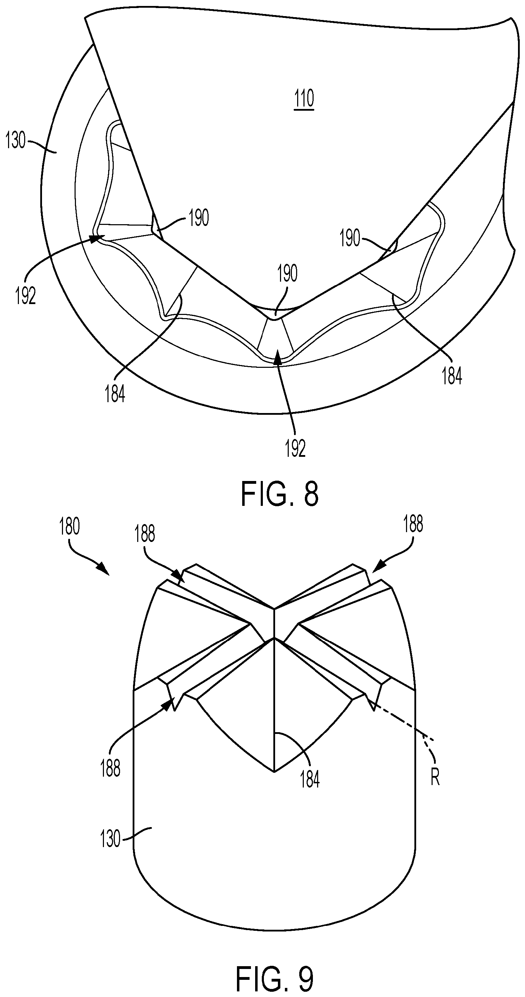

Suction can be continued until a sufficient amount of the saline has been removed from the trocar to clear the channels 134A, 134B. When cleaning is complete, the valve 120 is turned to block the inlet (as shown in Figure 6). The instrument 110 can then be advanced proximally through the seal 130 to continue operation at the surgical site, as shown in Figure 8. This view shows the instrument 110 pushed through the access valve 130 to pass distally beyond the trocar into the surgical site.

In an embodiment, the access valve or seal 130 has a contoured design that enables smoke particles to be removed from the surgical site even during active operation of the instrument 110, between cleaning sessions. In particular, when the seal is flexed open as shown in Figure 8, it forms small openings 190 between the seal 130 and the instrument 110. These openings allow smoke particles to escape into the trocar, where they are drawn into the channel 134A and through gas exit path P2 toward the filter 122.

Figures 9-11 show additional elements of the access valve 130, according to an embodiment. The access valve 130 includes a distal face 180 that faces in a distal direction inside the trocar, toward the surgical site. The access valve 130 includes two intersecting slits 182 that divide the face into four petals or leaflets 186A, 186B, 186C, and 186D. The slits 182 pass through the face to allow the seal to open. The petals 186A, B, C, D each have a crease or fold 184 in the middle of the petal, between the slits forming each petal. When a medical instrument pushes on the face from a proximal side, the petals bend to open at the slits. As the petals bend open, the crease 184 of each petal contacts and slides against the instrument 110. This is shown in the view of Figure 8. In an embodiment, the access valve or seal 130 is made of a flexible material such as silicon rubber. The four petals are resilient and bend to open at the intersecting first and second slits upon pressure applied to the petals in a distal direction.

In an embodiment, the intersecting slits 182 are positioned in recesses 188 in the distal face of the seal. The recesses 188 comprise two intersecting valleys or trenches crossing the face in a radial direction R, along the radius of the face toward the periphery. These recesses act to place the slits 182 away from the most distal surface of the face 180, recessing the slits into the valleys. The face 180 of the access valve has a three-dimensional contour including the creases 184, recesses 188, and slits 182. This contour is designed to enable the valve to seal completely when the instrument is withdrawn into the trocar but also to flex open in very intentional ways when the instrument is advanced forward into the surgical site.

When an instrument 110 pushes the slits 182 open as shown in Figure 8, the valleys 188 create the openings 190 between the petals and the instrument. The petals flex to open in response to passage of a medical instrument through the access valve in a distal direction, and the shape of the valleys creates gas pathway chutes 192 along the petals. Gas such as smoke can exit the surgical site through these openings 190 into the chutes 192. The openings 190 form at the corner of each petal, where the valleys 188 are positioned. In the embodiment where the access valve has two intersecting slits and four petals, four openings 190 are formed.

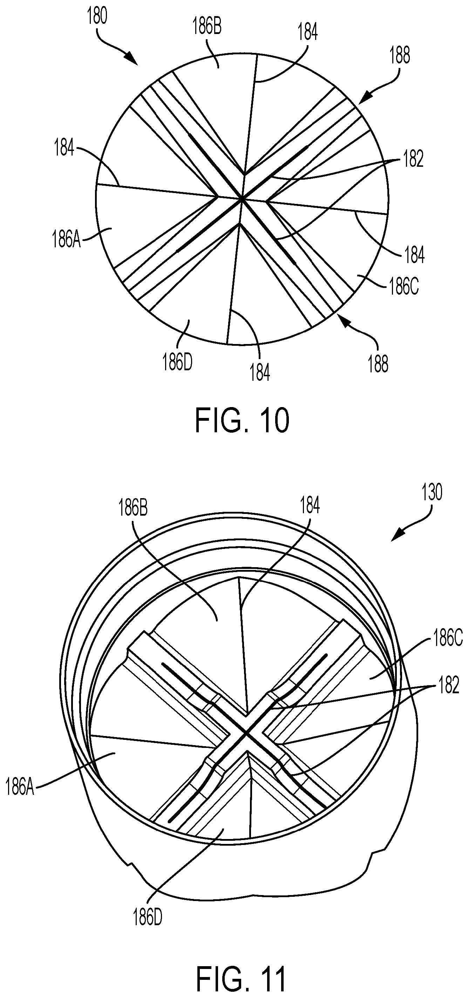

A lower view of the face 180 of the access valve is shown in Figure 10. This figure shows the face 180 with four petals 186A, B, C, D, each petal formed in a quadrant of the valve. The slits 182 intersect in a cross or X-shape, forming the petals. The slits 182 are placed at the bottom of the valleys or recesses which extend across the face of the valve.

A top perspective view of the access valve 130 is shown in Figure 11, showing the reverse side of the seal as seen from inside the trocar. The instrument 110 pushes down on this side of the valve 130 when the instrument is advanced forward into the surgical site. The slits 182 can be seen in the recessed valleys, and the creases 184 are shown in the middle of each petal.

Gas and smoke can accumulate in a surgical site during a surgical procedure due to the energy applied by surgical tools to tissue. This smoke accumulation creates a pressure within the body cavity, such as the abdomen or pneumoperitoneum. The openings 190 relieve this pressure, enabling the gas or smoke to move toward the lower pressure atmosphere of the ambient environment. The smoke will move through the openings 190 and through the first channel 134A in the trocar toward the filter 122. At this point, the suction supply has been discontinued because cleaning is not taking place, so the second channel 134B will be blocked by the suction valve 121 and will not pass to ambient air.

The filter 122 is designed to facilitate smoke removal during surgery. The size of the filter cartridge 122, filter medium 122B, and openings 122C will influence the rate of evacuation of smoke from the surgical site. The filter rate relieves intra-abdominal pressure, improves the surgical view, and facilitates smooth operation of the surgery. The filter also helps maintain the cleanliness of the operating room air for the safety of the surgical team. In an embodiment, the filter has a length (along path P2) of 10-30mm (such as 10, 15, 20, 25, or 30mm or sizes between those) and a diameter of 10-14mm (such as 10, 11, 12, 13, or 14mm or sizes between those). In an embodiment, the openings 122C are equally sized perforations arranged in radial lines across the rear face of the filter.

The filter 122 is blocked by the valve 120 during cleaning, so the filter function is temporarily discontinued during cleaning. However the cleaning operation can be quite short, such as a few seconds (2, 3, 4, 5, 6, 7, 8 seconds). Because the interruption in filtering is short, it should not cause a significant increase in gas pressure within the body cavity. Also, during cleaning, any gas or smoke particles that have entered the trocar body through the openings 190 just prior to cleaning can be washed away with the saline spray and suction.

The access valve or seal 130 thus provides two functions - sealing the instrument inside the trocar when the instrument is withdrawn proximally for cleaning, as well as forming smoke evacuation channels when the instrument is extended distally for operating. The access valve 130 is a one-way valve allowing passage of the instrument in one direction (distally), while also enabling retrograde flow of smoke (proximally). The contoured access valve 130 with recesses 188 and the multiport valve 120 enable the trocar 112 to provide bidirectional flow through the channel 134A. When the seal 130 is closed for cleaning, fluid flows distally through the channel 134A. When the seal 130 is open for smoke filtering, smoke and gas particles flow proximally through the same channel 134A.

The channel 134A acts as a bidirectional channel. When the multi-port valve is in the first position, the fluid flow path is formed through the first channel in a distal direction. When the multi-port valve is in the second position, the gas exit path is formed through the first channel in a proximal direction.

The trocar 112 enables both instrument cleaning and smoke filtering in vivo without removing the instrument from the trocar and without exposing the filter to cleaning liquids, thus maintaining the function of the cleaning and filtering, improving the surgical view, and improving the experience for both the surgical team and patient.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

In example 1, a surgical access device comprises a trocar comprising a proximal housing comprising a first port and a second port; an elongated shaft extending from the proximal housing to a distal end; a first channel and a second channel formed in the elongated shaft; and an access valve at the distal end of the elongated shaft; and a multiport valve fitted to the first port, where the multiport valve comprises an inlet, an outlet, a central branch, and a valve stem rotatable between a first position exposing the central branch to the inlet and blocking the outlet, and a second position exposing the central branch to the outlet and blocking the inlet, wherein the access valve comprises a flexible face comprising four petals divided by a first slit and a second slit intersecting the first slit, wherein the first slit is positioned in a first recess in the face and the second slit is positioned in a second recess in the face, the first and second recesses forming openings when the access valve is flexed open.

Example 2 comprises the device of example 1, wherein the central branch of the multiport valve communicates with the first channel in the elongated shaft of the trocar.

Example 3 comprises the device of example 2, wherein the second port of the trocar communicates with the second channel in the elongated shaft of the trocar.

Example 4 comprises the device of any precedent example, further comprising a suction valve fitted to the second port.

Example 5 comprises the device of any precedent example, further comprising a filter connected to the outlet of the multiport valve.

Example 6 comprises the device of any precedent example, wherein the first channel is bi-directional.

Example 7 comprises the device of example 6, wherein when the multiport valve is in the first position, a fluid flow path is formed through the first channel in a distal direction; and when the multiport valve is in the second position, a gas exit path is formed through the first channel in a proximal direction.

Example 8 comprises the device of any preceding example, wherein the four petals are resilient and bend to open at the intersecting first and second slits upon pressure applied to the petals in a distal direction.

Example 9 comprises the device of example 8, wherein the first and second recesses in the face comprise intersecting valleys crossing the face in a radial direction.

Example 10 comprises the device of example 9, wherein the intersecting valleys form gas pathway chutes when the petals bend open.

Example 11 comprises the device of any preceding example, further comprising a cannula fitted over the elongated shaft.

Example 12 comprises the device of any preceding example, wherein the inlet of the multiport valve interfaces with a saline supply, and wherein the second port of the trocar interfaces with a suction supply.

Example 13 comprises the device of example 12, wherein when the multiport valve is in the first position, the inlet of the multiport valve communicates with the first channel to supply the saline through the first channel in a distal direction.

Example 14 comprises the device of example 13, wherein when the multiport valve is in the second position, the outlet of the multiport valve communicates with the first channel to release gas through the first channel in a proximal direction.

Example 15 comprises the device of any preceding example, wherein when the multiport valve is positioned in a closed position, a side wall of the valve stem blocks both the inlet and the outlet.

In example 16, a surgical access device comprises a trocar comprising a proximal housing comprising a valve port and a suction port; an elongated shaft extending from the proximal housing to a distal end; a cannula fitted over the elongated shaft; a central lumen passing through the elongated shaft; a first channel and a second channel between the elongated shaft and the cannula; and an access valve at the distal end of the elongated shaft; a multiport valve fitted to the valve port, where the multiport valve comprises an inlet, an outlet, a central branch, and a valve stem rotatable between a first position exposing the central branch to the inlet and blocking the outlet, and a second position exposing the central branch to the outlet and blocking the inlet; and a filter mated with the outlet of the multiport valve, wherein the access valve comprises a face comprising petals divided by a first slit and a second slit intersecting the first slit, and wherein the petals flex to form gas pathway chutes in response to passage of a medical instrument through the access valve in a distal direction.

Example 17 comprises the device of example 16, wherein the first slit is positioned in a first recess in the face and the second slit is positioned in a second recess in the face.

Example 18 comprises the device of example 16, wherein the first and second recesses form intersecting valleys across the face of the access valve.

Example 19 comprises the device of example 16, wherein the inlet of the multiport valve interfaces with a saline supply, and wherein the suction port of the trocar interfaces with a suction supply.

Example 20 comprises the device of example 19, wherein when the multiport valve is in the first position, the inlet of the multiport valve communicates with the first channel to supply saline through the first channel in a distal direction, and wherein when the multiport valve is in the second position, the outlet of the multiport valve communicates with the first channel to release gas through the first channel in a proximal direction.

## Claims

1. A surgical access device comprising:
a trocar comprising:
a proximal housing comprising a first port and a second port;
an elongated shaft extending from the proximal housing to a distal end;
a first channel and a second channel formed in the elongated shaft; and
an access valve at the distal end of the elongated shaft; and
a multiport valve fitted to the first port, where the multiport valve comprises an inlet, an outlet, a central branch, and a valve stem rotatable between a first position exposing the central branch to the inlet and blocking the outlet, and a second position exposing the central branch to the outlet and blocking the inlet,
wherein the access valve comprises a flexible face comprising four petals divided by a first slit and a second slit intersecting the first slit, wherein the first slit is positioned in a first recess in the face and the second slit is positioned in a second recess in the face, the first and second recesses forming openings when the access valve is flexed open.

2. The device of claim 1, wherein the central branch of the multiport valve communicates with the first channel in the elongated shaft of the trocar.

3. The device of claim 2, wherein the second port of the trocar communicates with the second channel in the elongated shaft of the trocar.

4. The device of any precedent claim, further comprising a suction valve fitted to the second port.

5. The device of any precedent claim, further comprising a filter connected to the outlet of the multiport valve.

6. The device of claim any precedent claim, wherein the first channel is bi-directional.

7. The device of claim 6, wherein when the multiport valve is in the first position, a fluid flow path is formed through the first channel in a distal direction; and when the multiport valve is in the second position, a gas exit path is formed through the first channel in a proximal direction.

8. The device of claim any preceding claim, wherein the four petals are resilient and bend to open at the intersecting first and second slits upon pressure applied to the petals in a distal direction.

9. The device of claim 8, wherein the first and second recesses in the face comprise intersecting valleys crossing the face in a radial direction.

10. The device of claim 9, wherein the intersecting valleys form gas pathway chutes when the petals bend open.

11. The device of any preceding claim, further comprising a cannula fitted over the elongated shaft.

12. The device of any preceding claim, wherein the inlet of the multiport valve interfaces with a saline supply, and wherein the second port of the trocar interfaces with a suction supply.

13. The device of claim 12, wherein when the multiport valve is in the first position, the inlet of the multiport valve communicates with the first channel to supply the saline through the first channel in a distal direction.

14. The device of claim 13, wherein when the multiport valve is in the second position, the outlet of the multiport valve communicates with the first channel to release gas through the first channel in a proximal direction.

15. The device of any preceding claim, wherein when the multiport valve is positioned in a closed position, a side wall of the valve stem blocks both the inlet and the outlet.
